# EUROPEAN PATENT APPLICATION

(11) **EP 1 002 863 A1**
(43) Date of publication of application: **24.05.2000**
(21) Application number: 98402540.3
(22) Date of filing: 13.10.1998
(51) Int. Cl.: C12N 15/12, C07K 14/705, C07K 16/28, G01N 33/566

(54) **A potassium channel member of the erg family**

(71) Applicant: Sanofi-Synthélabo, 75013 Paris (FR)
(72) Inventor: Renard, Stéphane, 92500 Rueil Malmaison (FR); Avenet, Patrick, 91120 Palaiseau (FR)
(74) Representative: Desaix, Anne

(57) **Abstract**

herg4 polypeptides and polynucleotides and methods for producing such polypeptides by recombinant techniques are disclosed. Also disclosed are methods for utilizing herg4 polypeptides and polynucleotides in the design of protocols for the treatment of (i) dysfunctions or diseases, including, but not limited to, epilepsy, migraine, cell proliferation, comportemental troubles, among others and diagnostic assays for such conditions.

SEQUENCE LISTING

(1) GENERAL INFORMATION:

(i) APPLICANT:
(A) NAME: SYNTHELABO
(B) STREET: 22 avenue Galilee
(C) CITY: LE PLESSIS-ROBINSON
(E) COUNTRY: FRANCE
(F) POSTAL CODE (ZIP): 92350
(G) TELEPHONE: (33) 1 45 37 55 55

(ii) TITLE OF INVENTION: erg4 new putative potassium channel

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 3252 base pairs
(B) TYPE: nucleic acid
(C) STRANDEDNESS: double
(D) TOPOLOGY: circular

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens

(ix) FEATURE:
(A) NAME/KEY: CDS
(B) LOCATION:1..3249

(xi) SEQUENCE DESCRIPTION: SEQ ID NO: 1:

ATG CCG GCC ATG CGG GGC CTC CTG GCG CCG CAG AAC ACC TTC CTG GAC 48
Met Pro Ala Met Arg Gly Leu Leu Ala Pro Gln Asn Thr Phe Leu Asp
1               5                   10                  15

ACC ATC GCT ACG CGC TTC GAC GGC ACG CAC AGT AAC TTC GTG CTG GGC 96
Thr Ile Ala Thr Arg Phe Asp Gly Thr His Ser Asn Phe Val Leu Gly
            20                  25                  30

AAC GCC CAG GTG GCG GGG CTC TTC CCC GTG GTC TAC TGC TCT GAT GGC 144
Asn Ala Gln Val Ala Gly Leu Phe Pro Val Val Tyr Cys Ser Asp Gly
        35                  40                  45

TTC TGT GAC CTC ACG GGC TTC TCC CGG GCT GAG GTC ATG CAG CGG GGC 192
Phe Cys Asp Leu Thr Gly Phe Ser Arg Ala Glu Val Met Gln Arg Gly
    50                  55                  60

TGT GCC TGC TCC TTC CTT TAT GGG CCA GAC ACC AGT GAG CTC GTC CGC 240
Cys Ala Cys Ser Phe Leu Tyr Gly Pro Asp Thr Ser Glu Leu Val Arg
65                  70                  75                  80

CAA CAG ATC CGC AAG GCC CTG GAC GAG CAC AAG GAG TTC AAG GCT GAG 288
Gln Gln Ile Arg Lys Ala Leu Asp Glu His Lys Glu Phe Lys Ala Glu
                85                  90                  95

CTG ATC CTG TAC CGG AAG AGC GGG CTC CCG TTC TGG TGT CTC CTG GAT 336
Leu Ile Leu Tyr Arg Lys Ser Gly Leu Pro Phe Trp Cys Leu Leu Asp
            100                 105                 110

GTG ATA CCC ATA AAG AAT GAG AAA GGG GAG GTG GCT CTC TTC CTA GTC 384
Val Ile Pro Ile Lys Asn Glu Lys Gly Glu Val Ala Leu Phe Leu Val
        115                 120                 125

TCT CAC AAG GAC ATC AGC GAA ACC AAG AAC CGA GGG GGC CCC GAC AGA 432
Ser His Lys Asp Ile Ser Glu Thr Lys Asn Arg Gly Gly Pro Asp Arg
    130                 135                 140

TGG AAG GAG ACA GGT GGT GGC CGG CGC CGA TAT GGC CGG GCA CGA TCC 480
Trp Lys Glu Thr Gly Gly Gly Arg Arg Arg Tyr Gly Arg Ala Arg Ser
145                 150                 155                 160

AAA GGC TTC AAT GCC AAC CGG CGG CGG AGC CGG GCC GTG CTC TAC CAC 528
Lys Gly Phe Asn Ala Asn Arg Arg Arg Ser Arg Ala Val Leu Tyr His
                165                 170                 175

CTG TCC GGG CAC CTG CAG AAG CAG CCC AAG GGC AAG CAC AAG CTC AAT 576
Leu Ser Gly His Leu Gln Lys Gln Pro Lys Gly Lys His Lys Leu Asn
            180                 185                 190

AAG GGG GTG TTT GGG GAG AAA CCA AAC TTG CCT GAG TAC AAA GTA GCC 624
Lys Gly Val Phe Gly Glu Lys Pro Asn Leu Pro Glu Tyr Lys Val Ala
        195                 200                 205

GCC ATC CGG AAG TCG CCC TTC ATC CTG TTG CAC TGT GGG GCA CTG AGA 672
Ala Ile Arg Lys Ser Pro Phe Ile Leu Leu His Cys Gly Ala Leu Arg
    210                 215                 220

GCC ACC TGG GAT GGC TTC ATC CTG CTC GCC ACA CTC TAT GTG GCT GTC 720
Ala Thr Trp Asp Gly Phe Ile Leu Leu Ala Thr Leu Tyr Val Ala Val
225                 230                 235                 240

ACT GTG CCC TAC AGC GTG TGT GTG AGC ACA GCA CGG GAG CCC AGT GCC 768
Thr Val Pro Tyr Ser Val Cys Val Ser Thr Ala Arg Glu Pro Ser Ala
                245                 250                 255

GCC CGC GGC CCG CCC AGC GTC TGT GAC CTG GCC GTG GAG GTC CTC TTC 816
Ala Arg Gly Pro Pro Ser Val Cys Asp Leu Ala Val Glu Val Leu Phe
            260                 265                 270

ATC CTT GAC ATT GTG CTG AAT TTC CGT ACC ACA TTC GTG TCC AAG TCG 864
Ile Leu Asp Ile Val Leu Asn Phe Arg Thr Thr Phe Val Ser Lys Ser
        275                 280                 285

GGC CAG GTG GTG TTT GCC CCA AAG TCC ATT TGC CTC CAC TAC GTC ACC 912
Gly Gln Val Val Phe Ala Pro Lys Ser Ile Cys Leu His Tyr Val Thr
    290                 295                 300

ACC TGG TTC CTG CTG GAT GTC ATC GCG GCG CTG CCC TTT GAC CTG CTA 960
Thr Trp Phe Leu Leu Asp Val Ile Ala Ala Leu Pro Phe Asp Leu Leu
305                 310                 315                 320

CAT GCC TTC AAG GTC AAC GTG TAC TTC GGG GCC CAT CTG CTG AAG ACG 1008
His Ala Phe Lys Val Asn Val Tyr Phe Gly Ala His Leu Leu Lys Thr
                325                 330                 335

GTG CGC CTG CTG CGC CTG CTG CGC CTG CTT CCG CGG CTG GAC CGG TAC 1056
Val Arg Leu Leu Arg Leu Leu Arg Leu Leu Pro Arg Leu Asp Arg Tyr
            340                 345                 350

TCG CAG TAC AGC GCC GTG GTG CTG ACA CTG CTC ATG GCC GTG TTC GCC 1104
Ser Gln Tyr Ser Ala Val Val Leu Thr Leu Leu Met Ala Val Phe Ala
        355                 360                 365

CTG CTC GCG CAC TGG GTC GCC TGC GTC TGG TTT TAC ATT GGC CAG CGG 1152
Leu Leu Ala His Trp Val Ala Cys Val Trp Phe Tyr Ile Gly Gln Arg
    370                 375                 380

GAG ATC GAG AGC AGC GAA TCC GAG CTG CCT GAG ATT GGC TGG CTG CAG 1200
Glu Ile Glu Ser Ser Glu Ser Glu Leu Pro Glu Ile Gly Trp Leu Gln
385                 390                 395                 400

GAG CTG GCC CGC CGA CTG GAG ACT CCC TAC TAC CTG GTG GGC CGG AGG 1248
Glu Leu Ala Arg Arg Leu Glu Thr Pro Tyr Tyr Leu Val Gly Arg Arg
                405                 410                 415

CCA GCT GGA GGG AAC AGC TCC GGC CAG AGT GAC AAC TGC AGC AGC AGC 1296
Pro Ala Gly Gly Asn Ser Ser Gly Gln Ser Asp Asn Cys Ser Ser Ser
            420                 425                 430

AGC GAG GCC AAC GGG ACG GGG CTG GAG CTG CTG GGC GGC CCG TCG CTG 1344
Ser Glu Ala Asn Gly Thr Gly Leu Glu Leu Leu Gly Gly Pro Ser Leu
        435                 440                 445

CGC AGC GCC TAC ATC ACC TCC CTC TAC TTC GCA CTC AGC AGC CTC ACC 1392
Arg Ser Ala Tyr Ile Thr Ser Leu Tyr Phe Ala Leu Ser Ser Leu Thr
    450                 455                 460

AGC GTG GGC TTC GGC AAC GTG TCC GCC AAC ACG GAC ACC GAG AAG ATC 1440
Ser Val Gly Phe Gly Asn Val Ser Ala Asn Thr Asp Thr Glu Lys Ile
465                 470                 475                 480

TTC TCC ATC TGC ACC ATG CTC ATC GGC GCC CTG ATG CAC GCG GTG GTG 1488
Phe Ser Ile Cys Thr Met Leu Ile Gly Ala Leu Met His Ala Val Val
                485                 490                 495

TTT GGG AAC GTG ACG GCC ATC ATC CAG CGC ATG TAC GCC CGC CGC TTT 1536
Phe Gly Asn Val Thr Ala Ile Ile Gln Arg Met Tyr Ala Arg Arg Phe
            500                 505                 510

CTG TAC CAC AGC CGC ACG CGC GAC CTG CGC GAC TAC ATC CGC ATC CAC 1584
Leu Tyr His Ser Arg Thr Arg Asp Leu Arg Asp Tyr Ile Arg Ile His
        515                 520                 525

CGT ATC CCC AAG CCC CTC AAG CAG CGC ATG CTG GAG TAC TTC CAG GCC 1632
Arg Ile Pro Lys Pro Leu Lys Gln Arg Met Leu Glu Tyr Phe Gln Ala
    530                 535                 540

ACC TGG GCG GTG AAC AAT GGC ATC GAC ACC ACC GAG CTG CTG CAG AGC 1680
Thr Trp Ala Val Asn Asn Gly Ile Asp Thr Thr Glu Leu Leu Gln Ser
545                 550                 555                 560

CTC CCT GAC GAG CTG CGC GCA GAC ATC GCC ATG CAC CTG CAC AAG GAG 1728
Leu Pro Asp Glu Leu Arg Ala Asp Ile Ala Met His Leu His Lys Glu
                565                 570                 575

GTC CTG CAG CTG CCA CTG TTT GAG GCG GCC AGC CGC GGC TGC CTG CGG 1776
Val Leu Gln Leu Pro Leu Phe Glu Ala Ala Ser Arg Gly Cys Leu Arg
            580                 585                 590

GCA CTG TCT CTG GCC CTG CGG CCC GCC TTC TGC ACG CCG GGC GAG TAC 1824
Ala Leu Ser Leu Ala Leu Arg Pro Ala Phe Cys Thr Pro Gly Glu Tyr
        595                 600                 605

CTC ATC CAC CAA GGC GAT GCC CTG CAG GCC CTC TAC TTT GTC TGC TCT 1872
Leu Ile His Gln Gly Asp Ala Leu Gln Ala Leu Tyr Phe Val Cys Ser
    610                 615                 620

GGC TTC ATG GAG GTG CTC AAG GGT GGC ACC GTG CTC GCC ATC CTA GGG 1920
Gly Phe Met Glu Val Leu Lys Gly Gly Thr Val Leu Ala Ile Leu Gly
625                 630                 635                 640

AAG GGC GAC CTG ATC GGC TGT GAG CTG CCC CGG CGG GAG CAG GTG GTA 1968
Lys Gly Asp Leu Ile Gly Cys Glu Leu Pro Arg Arg Glu Gln Val Val
                645                 650                 655

AAG GCC AAT GCC GAC GTG AAG GGG CTG ACG TAC TGC GTC CTG CAG TGT 2016
Lys Ala Asn Ala Asp Val Lys Gly Leu Thr Tyr Cys Val Leu Gln Cys
            660                 665                 670

CTG CAG CTG GCT GGC CTG CAC GAC AGC CTT GCG CTG TAC CCC GAG TTT 2064
Leu Gln Leu Ala Gly Leu His Asp Ser Leu Ala Leu Tyr Pro Glu Phe
        675                 680                 685

GCC CCG CGC TTC AGT CGT GGC CTC CGA GGG GAG CTC AGC TAC AAC CTG 2112
Ala Pro Arg Phe Ser Arg Gly Leu Arg Gly Glu Leu Ser Tyr Asn Leu
    690                 695                 700

GGT GCT GGG GGA GGC TCT GCA GAG GTG GAC ACC AGC TCC CTG AGC GGC 2160
Gly Ala Gly Gly Gly Ser Ala Glu Val Asp Thr Ser Ser Leu Ser Gly
705                 710                 715                 720

GAC AAT ACC CTT ATG TCC ACG CTG GAG GAG AAG GAG ACA GAT GGG GAG 2208
Asp Asn Thr Leu Met Ser Thr Leu Glu Glu Lys Glu Thr Asp Gly Glu
                725                 730                 735

CAG GGC CCC ACG GTC TCC CCA GCC CCA GCT GAT GAG CCC TCC AGC CCC 2256
Gln Gly Pro Thr Val Ser Pro Ala Pro Ala Asp Glu Pro Ser Ser Pro
            740                 745                 750

CTG CTG TCC CCT GGC TGC ACC TCC TCA TCC TCA GCT GCC AAG CTG CTA 2304
Leu Leu Ser Pro Gly Cys Thr Ser Ser Ser Ser Ala Ala Lys Leu Leu
        755                 760                 765

TCC CCA CGT CGA ACA GCA CCC CGG CCT CGT CTA GGT GGC AGA GGG AGG 2352
Ser Pro Arg Arg Thr Ala Pro Arg Pro Arg Leu Gly Gly Arg Gly Arg
    770                 775                 780

CCA GGC AGG GCA GGG GCT TTG AAG GCT GAG GCT GGC CCC TCT GCT CCC 2400
Pro Gly Arg Ala Gly Ala Leu Lys Ala Glu Ala Gly Pro Ser Ala Pro
785                 790                 795                 800

CCA CGG GCC CTA GAG GGG CTA CGG CTG CCC CCC ATG CCA TGG AAT GTG 2448
Pro Arg Ala Leu Glu Gly Leu Arg Leu Pro Pro Met Pro Trp Asn Val
                805                 810                 815

CCC CCA GAT CTG AGC CCC AGG GTA GTA GAT GGC ATT GAA GAC GGC TGT 2496
Pro Pro Asp Leu Ser Pro Arg Val Val Asp Gly Ile Glu Asp Gly Cys
            820                 825                 830

GGC TCG GAC CAG CCC AAG TTC TCT TTC CGC GTG GGC CAG TCT GGC CCG 2544
Gly Ser Asp Gln Pro Lys Phe Ser Phe Arg Val Gly Gln Ser Gly Pro
        835                 840                 845

GAA TGT AGC AGC AGC CCC TCC CCT GGA CCA GAG AGC GGC CTG CTC ACT 2592
Glu Cys Ser Ser Ser Pro Ser Pro Gly Pro Glu Ser Gly Leu Leu Thr
    850                 855                 860

GTT CCC CAT GGG CCC AGC GAG GCA AGG AAC ACA GAC ACA CTG GAC AAG 2640
Val Pro His Gly Pro Ser Glu Ala Arg Asn Thr Asp Thr Leu Asp Lys
865                 870                 875                 880

CTT CGG CAG GCG GTG ACA GAG CTG TCA GAG CAG GTG CTG CAG ATG CGG 2688
Leu Arg Gln Ala Val Thr Glu Leu Ser Glu Gln Val Leu Gln Met Arg
                885                 890                 895

GAA GGA CTG CAG TCA CTT CGC CAG GCT GTG CAG CTT GTC CTG GCG CCC 2736
Glu Gly Leu Gln Ser Leu Arg Gln Ala Val Gln Leu Val Leu Ala Pro
            900                 905                 910

CAC AGG GAG GGT CCG TGC CCT CGG GCA TCG GGA GAG GGG CCG TGC CCA 2784
His Arg Glu Gly Pro Cys Pro Arg Ala Ser Gly Glu Gly Pro Cys Pro
        915                 920                 925

GCC AGC ACC TCC GGG CTT CTG CAG CCT CTG TGT GTG GAC ACT GGG GCA 2832
Ala Ser Thr Ser Gly Leu Leu Gln Pro Leu Cys Val Asp Thr Gly Ala
    930                 935                 940

TCC TCC TAC TGC CTG CAG CCC CCA GCT GGC TCT GTC TTG AGT GGG ACT 2880
Ser Ser Tyr Cys Leu Gln Pro Pro Ala Gly Ser Val Leu Ser Gly Thr
945                 950                 955                 960

TGG CCC CAC CCT CGT CCG GGG CCT CCT CCC CTC ATG GCA CCC TGG CCC 2928
Trp Pro His Pro Arg Pro Gly Pro Pro Pro Leu Met Ala Pro Trp Pro
                965                 970                 975

TGG GGT CCC CCA GCG TCT CAG AGC TCC CCC TGG CCT CGA GCC ACA GCT 2976
Trp Gly Pro Pro Ala Ser Gln Ser Ser Pro Trp Pro Arg Ala Thr Ala
            980                 985                 990

TTC TGG ACC TCC ACC TCA GAC TCA GAG CCC CCT GCC TCA GGA GAC CTC 3024
Phe Trp Thr Ser Thr Ser Asp Ser Glu Pro Pro Ala Ser Gly Asp Leu
        995                 1000                1005

TGC TCT GAG CCC AGC ACC CCT GCC TCC CCT CCT CCT TCT GAG GAA GGG 3072
Cys Ser Glu Pro Ser Thr Pro Ala Ser Pro Pro Pro Ser Glu Glu Gly
    1010                1015                1020

GCT AGG ACT GGG CCC GCA GAG CCT GTG AGC CAG GCT GAG GCT ACC AGC 3120
Ala Arg Thr Gly Pro Ala Glu Pro Val Ser Gln Ala Glu Ala Thr Ser
1025                1030                1035                1040

ACT GGA GAG CCC CCA CCA GGG TCA GGG GGC CTG GCC TTG CCC TGG GAC 3168
Thr Gly Glu Pro Pro Pro Gly Ser Gly Gly Leu Ala Leu Pro Trp Asp
                1045                1050                1055

CCC CAC AGC CTG GAG ATG GTG CTT ATT GGC TGC CAT GGC TCT GGC ACA 3216
Pro His Ser Leu Glu Met Val Leu Ile Gly Cys His Gly Ser Gly Thr
            1060                1065                1070

GTC CAG TGG ACC CAG GAA GAA GGC ACA GGG GTC TGA 3252
Val Gln Trp Thr Gln Glu Glu Gly Thr Gly Val
        1075                1080

(2) INFORMATION FOR SEQ ID NO: 2:

(i) SEQUENCE CHARACTERISTICS:
(A) LENGTH: 1083 amino acids
(B) TYPE: amino acid
(D) TOPOLOGY: linear

(ii) MOLECULE TYPE: protein
(xi) SEQUENCE DESCRIPTION: SEQ ID NO: 2:

Met Pro Ala Met Arg Gly Leu Leu Ala Pro Gln Asn Thr Phe Leu Asp
1               5                   10                  15

Thr Ile Ala Thr Arg Phe Asp Gly Thr His Ser Asn Phe Val Leu Gly
            20                  25                  30

Asn Ala Gln Val Ala Gly Leu Phe Pro Val Val Tyr Cys Ser Asp Gly
        35                  40                  45

Phe Cys Asp Leu Thr Gly Phe Ser Arg Ala Glu Val Met Gln Arg Gly
    50                  55                  60

Cys Ala Cys Ser Phe Leu Tyr Gly Pro Asp Thr Ser Glu Leu Val Arg
65                  70                  75                  80

Gln Gln Ile Arg Lys Ala Leu Asp Glu His Lys Glu Phe Lys Ala Glu
                85                  90                  95

Leu Ile Leu Tyr Arg Lys Ser Gly Leu Pro Phe Trp Cys Leu Leu Asp
            100                 105                 110

Val Ile Pro Ile Lys Asn Glu Lys Gly Glu Val Ala Leu Phe Leu Val
        115                 120                 125

Ser His Lys Asp Ile Ser Glu Thr Lys Asn Arg Gly Gly Pro Asp Arg
    130                 135                 140

Trp Lys Glu Thr Gly Gly Gly Arg Arg Arg Tyr Gly Arg Ala Arg Ser
145                 150                 155                 160

Lys Gly Phe Asn Ala Asn Arg Arg Arg Ser Arg Ala Val Leu Tyr His
                165                 170                 175

Leu Ser Gly His Leu Gln Lys Gln Pro Lys Gly Lys His Lys Leu Asn
            180                 185                 190

Lys Gly Val Phe Gly Glu Lys Pro Asn Leu Pro Glu Tyr Lys Val Ala
        195                 200                 205

Ala Ile Arg Lys Ser Pro Phe Ile Leu Leu His Cys Gly Ala Leu Arg
    210                 215                 220

Ala Thr Trp Asp Gly Phe Ile Leu Leu Ala Thr Leu Tyr Val Ala Val
225                 230                 235                 240

Thr Val Pro Tyr Ser Val Cys Val Ser Thr Ala Arg Glu Pro Ser Ala
                245                 250                 255

Ala Arg Gly Pro Pro Ser Val Cys Asp Leu Ala Val Glu Val Leu Phe
            260                 265                 270

Ile Leu Asp Ile Val Leu Asn Phe Arg Thr Thr Phe Val Ser Lys Ser
        275                 280                 285

Gly Gln Val Val Phe Ala Pro Lys Ser Ile Cys Leu His Tyr Val Thr
    290                 295                 300

Thr Trp Phe Leu Leu Asp Val Ile Ala Ala Leu Pro Phe Asp Leu Leu
305                 310                 315                 320

His Ala Phe Lys Val Asn Val Tyr Phe Gly Ala His Leu Leu Lys Thr
                325                 330                 335

Val Arg Leu Leu Arg Leu Leu Arg Leu Leu Pro Arg Leu Asp Arg Tyr
            340                 345                 350

Ser Gln Tyr Ser Ala Val Val Leu Thr Leu Leu Met Ala Val Phe Ala
        355                 360                 365

Leu Leu Ala His Trp Val Ala Cys Val Trp Phe Tyr Ile Gly Gln Arg
    370                 375                 380

Glu Ile Glu Ser Ser Glu Ser Glu Leu Pro Glu Ile Gly Trp Leu Gln
385                 390                 395                 400

Glu Leu Ala Arg Arg Leu Glu Thr Pro Tyr Tyr Leu Val Gly Arg Arg
                405                 410                 415

Pro Ala Gly Gly Asn Ser Ser Gly Gln Ser Asp Asn Cys Ser Ser Ser
            420                 425                 430

Ser Glu Ala Asn Gly Thr Gly Leu Glu Leu Leu Gly Gly Pro Ser Leu
        435                 440                 445

Arg Ser Ala Tyr Ile Thr Ser Leu Tyr Phe Ala Leu Ser Ser Leu Thr
    450                 455                 460

Ser Val Gly Phe Gly Asn Val Ser Ala Asn Thr Asp Thr Glu Lys Ile
465                 470                 475                 480

Phe Ser Ile Cys Thr Met Leu Ile Gly Ala Leu Met His Ala Val Val
                485                 490                 495

Phe Gly Asn Val Thr Ala Ile Ile Gln Arg Met Tyr Ala Arg Arg Phe
            500                 505                 510

Leu Tyr His Ser Arg Thr Arg Asp Leu Arg Asp Tyr Ile Arg Ile His
        515                 520                 525

Arg Ile Pro Lys Pro Leu Lys Gln Arg Met Leu Glu Tyr Phe Gln Ala
    530                 535                 540

Thr Trp Ala Val Asn Asn Gly Ile Asp Thr Thr Glu Leu Leu Gln Ser
545                 550                 555                 560

Leu Pro Asp Glu Leu Arg Ala Asp Ile Ala Met His Leu His Lys Glu
                565                 570                 575

Val Leu Gln Leu Pro Leu Phe Glu Ala Ala Ser Arg Gly Cys Leu Arg
            580                 585                 590

Ala Leu Ser Leu Ala Leu Arg Pro Ala Phe Cys Thr Pro Gly Glu Tyr
        595                 600                 605

Leu Ile His Gln Gly Asp Ala Leu Gln Ala Leu Tyr Phe Val Cys Ser
    610                 615                 620

Gly Phe Met Glu Val Leu Lys Gly Gly Thr Val Leu Ala Ile Leu Gly
625                 630                 635                 640

Lys Gly Asp Leu Ile Gly Cys Glu Leu Pro Arg Arg Glu Gln Val Val
                645                 650                 655

Lys Ala Asn Ala Asp Val Lys Gly Leu Thr Tyr Cys Val Leu Gln Cys
            660                 665                 670

Leu Gln Leu Ala Gly Leu His Asp Ser Leu Ala Leu Tyr Pro Glu Phe
        675                 680                 685

Ala Pro Arg Phe Ser Arg Gly Leu Arg Gly Glu Leu Ser Tyr Asn Leu
    690                 695                 700

Gly Ala Gly Gly Gly Ser Ala Glu Val Asp Thr Ser Ser Leu Ser Gly
705                 710                 715                 720

Asp Asn Thr Leu Met Ser Thr Leu Glu Glu Lys Glu Thr Asp Gly Glu
                725                 730                 735

Gln Gly Pro Thr Val Ser Pro Ala Pro Ala Asp Glu Pro Ser Ser Pro
            740                 745                 750

Leu Leu Ser Pro Gly Cys Thr Ser Ser Ser Ser Ala Ala Lys Leu Leu
        755                 760                 765

Ser Pro Arg Arg Thr Ala Pro Arg Pro Arg Leu Gly Gly Arg Gly Arg
    770                 775                 780

Pro Gly Arg Ala Gly Ala Leu Lys Ala Glu Ala Gly Pro Ser Ala Pro
785                 790                 795                 800

Pro Arg Ala Leu Glu Gly Leu Arg Leu Pro Pro Met Pro Trp Asn Val
                805                 810                 815

Pro Pro Asp Leu Ser Pro Arg Val Val Asp Gly Ile Glu Asp Gly Cys
            820                 825                 830

Gly Ser Asp Gln Pro Lys Phe Ser Phe Arg Val Gly Gln Ser Gly Pro
        835                 840                 845

Glu Cys Ser Ser Ser Pro Ser Pro Gly Pro Glu Ser Gly Leu Leu Thr
    850                 855                 860

Val Pro His Gly Pro Ser Glu Ala Arg Asn Thr Asp Thr Leu Asp Lys
865                 870                 875                 880

Leu Arg Gln Ala Val Thr Glu Leu Ser Glu Gln Val Leu Gln Met Arg
                885                 890                 895

Glu Gly Leu Gln Ser Leu Arg Gln Ala Val Gln Leu Val Leu Ala Pro
            900                 905                 910

His Arg Glu Gly Pro Cys Pro Arg Ala Ser Gly Glu Gly Pro Cys Pro
        915                 920                 925

Ala Ser Thr Ser Gly Leu Leu Gln Pro Leu Cys Val Asp Thr Gly Ala
    930                 935                 940

Ser Ser Tyr Cys Leu Gln Pro Pro Ala Gly Ser Val Leu Ser Gly Thr
945                 950                 955                 960

Trp Pro His Pro Arg Pro Gly Pro Pro Pro Leu Met Ala Pro Trp Pro
                965                 970                 975

Trp Gly Pro Pro Ala Ser Gln Ser Ser Pro Trp Pro Arg Ala Thr Ala
            980                 985                 990

Phe Trp Thr Ser Thr Ser Asp Ser Glu Pro Pro Ala Ser Gly Asp Leu
        995                 1000                1005

Cys Ser Glu Pro Ser Thr Pro Ala Ser Pro Pro Pro Ser Glu Glu Gly
    1010                1015                1020

Ala Arg Thr Gly Pro Ala Glu Pro Val Ser Gln Ala Glu Ala Thr Ser
1025                1030                1035                1040

Thr Gly Glu Pro Pro Pro Gly Ser Gly Gly Leu Ala Leu Pro Trp Asp
                1045                1050                1055

Pro His Ser Leu Glu Met Val Leu Ile Gly Cys His Gly Ser Gly Thr
            1060                1065                1070

Val Gln Trp Thr Gln Glu Glu Gly Thr Gly Val
        1075                1080

## Description

### FIELD OF INVENTION

This invention relates to newly identified polynucleotides, polypeptides encoded by such polynucleotides, to the use of such polynucleotides and polypeptides and to their production. More particularly, the polynucleotides and polypeptides of the present invention relate to erg potassium channel family, hereinafter referred to as herg4 (Human Erg Related Gene 4). The invention also relates to inhibiting or activating the action of such polynucleotides and polypeptides.

### BACKGROUND OF THE INVENTION

Potassium channels are involved in the maintenance of the cell resting membrane potential, and particularly for excitable cells in the control of their excitability by maintening or allowing to recover the membrane potential. Potassium channels have also been involved in the regulation of cell proliferation.

This indicates that these channels have an interesting potential as therapeutic targets. Clearly there is a need for identification and characterization of further channels which can play a role in (i) preventing, ameliorating or correcting dysfunctions or diseases, including, but not limited to, epilepsy, migraine, cell proliferation, behavioural troubles and (ii) altering or preventing effect of endogenous neurotransmitters and hormones.

### SUMMARY OF THE INVENTION

In one aspect, the invention relates to herg4 polypeptides and recombinant materials and methods for their production. Another aspect of the invention relates to methods for using such herg4 polypeptides and polynucleotides. Such uses include the treatment of epilepsy, migraine, cell proliferation, comportemental troubles, to alter or prevent effect of endogenous neurotransmitters and hormones, or to diagnose or treat any disorder related to abnormal expression of these herg4 polypeptides, among others. In still another aspect, the invention relates to methods to identify agonists and antagonists using the materials provided by the invention, and treating conditions associated with herg4 imbalance with the identified compounds. Yet another aspect of the invention relates to diagnostic assays for detecting diseases associated with inappropriate herg4 activity or levels.

### DESCRIPTION OF THE INVENTION

### Definitions

The following definitions are provided to facilitate understanding of certain terms used frequently herein-below.

"herg4" refers, among others, to a polypeptide comprising the amino acid sequence set forth in SEQ ID NO:2, or an allelic variant thereof.

"Receptor Activity" or "Channel Activity" or "Biological Activity of the Receptor" or "Biological Activity of the Channel" refers to the metabolic or physiologic function of said herg4 including similar activities or improved activities or these activities with decreased undesirable side-effects. Also included are antigenic and immunogenic activities of said herg4.

"herg4 gene" refers to a polynucleotide comprising the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of a Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCG program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to herg4 polypeptides. The herg4 polypeptides include the polypeptide of SEQ ID NO:2, as well as polypeptides comprising the amino acid sequence of SEQ ID NO:2 , and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within herg4 polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% are highly preferred. Preferably herg4 polypeptides exhibit at least one biological activity of the receptor.

The herg4 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the herg4 polypeptides are also included in the invention. A fragment is a polypeptide having an amino acid sequence that entirely is the same as part, but not all, of the amino acid sequence of the aforementioned herg4 polypeptides. As with herg4 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of herg4 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of herg4 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate receptor or channel activity, including those with a similar activity or an improved activity, or with a decreased undesirable activity. Also included are those that are antigenic or immunogenic in an animal, especially in a human.

Preferably, all of these polypeptide fragments retain the biological activity of the receptor, including antigenic activity. Variants of the defined sequence and fragments also form part of the present invention. Preferred variants are those that vary from the referents by conservative amino acid substitutions i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The herg4 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to herg4 polynucleotides. herg4 polynucleotides include isolated polynucleotides which encode the herg4 polypeptides and fragments, and polynucleotides closely related thereto. More specifically, herg4 polynucleotides of the invention include a polynucleotide comprising the nucleotide sequence set forth in SEQ ID NO:1 encoding a herg4 polypeptide of SEQ ID NO: 2, and polynucleotide having the particular sequence of SEQ ID NO:1. herg4 polynucleotides further include a polynucleotide comprising a nucleotide sequence that has at least 80% identity to a nucleotide sequence encoding the herg4 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under herg4 polynucleotides are a nucleotide sequence which has sufficient identity to a nucleotide sequence contained in SEQ ID NO:1 or contained in the cDNA insert in the plasmid deposited with the ATCC Deposit number 203078 to hybridize under conditions useable for amplification or for use as a probe or marker. Moreover, herg4 polynucleotide includes nucleotide sequences having at least 80% identity to a nucleotide sequence encoding the herg4 polypeptide expressed by the cDNA insert deposited at the ATCC with Deposit Number 203078, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above herg4 polynucleotides.

A deposit containing a human herg4 cDNA has been deposited with the American Type Culture Collection (ATCC), 12301 Park Lawn Drive, Rockville, Maryland 20852, USA, on July 20, 1998, and assigned ATCC Deposit Number 203078. The deposited material (clone) is a DH5-a strain containing the expression vector pCR2.1 (Invitrogen) that further contains the herg4 cDNA, referred to as "herg4" upon deposit. The cDNA insert is within EcoRI site(s) in the vector. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure. The strain will be irrevocably and without restriction or condition released to the public upon the issuance of a patent.

herg4 of the invention is structurally related to other proteins of the potassium channel family, as shown by the results of sequencing the cDNA of SEQ ID NO:1 encoding human herg4. The cDNA sequence of SEQ ID NO:1 contains an open reading frame (nucleotide number 1 to 3252) encoding a polypeptide of 1084 amino acids of SEQ ID NO:2. Amino acid sequence of SEQ ID NO:2 has about 35.2%)identity (using BestFit version 1.0) in 885 amino acid residues with the drosophila elk protein (accession number: U04246). Nucleotide sequence of SEQ ID NO:1 has about 65% identity (using blastN version 1.4, GCG program package) in 2106 (197 to 2151) nucleotide residues with the elk cDNA (accession number: DMU04246).

One polynucleotide of the present invention encoding herg4 may be obtained using standard cloning and screening, from a cDNA library derived from mRNA in brain using the expressed sequence tag (EST) analysis (Adams, M.D., *et al. Science* (1991) 252:1651-1656; Adams, M.D. *et al., Nature,* (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding herg4 polypeptide of SEQ ID NO:2 may be identical to the polypeptide encoding sequence contained in SEQ ID NO:1 (nucleotide number 1 to 3252 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of herg4 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*.*, Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding herg4 variants comprising the amino acid sequence of herg4 polypeptide of SEQ ID NO:2 in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number 203078 or a fragment thereof, may be used as hybridization probes for cDNA and genomic DNA, to isolate full-length cDNAs and genomic clones encoding herg4 and to isolate cDNA and genomic clones of other genes that have a high sequence similarity to the herg4 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding herg4 polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease, either as a bulk composition or incorporated in a diagnostic kit.

### Vectors, Host Cells, Expression

The present invention also relates to a DNA or RNA molecule comprising an expression system, or vectors, wherein said expression system is capable of producing a herg4 polypeptide comprising an amino acid sequence, which has at least 80% identity with the polypeptide of SEQ ID NO: 2 when said expression system is present in a compatible host cell. It also relates to host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces and Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the herg4 polypeptide is to be expressed for use in screening assays, generally, it is preferred that the polypeptide be produced at the surface of the cell. In this event, the cells may be harvested prior to use in the screening assay. If herg4 polypeptide is secreted into the medium, the medium can be recovered in order to recover and purify the polypeptide; if produced intracellularly, the cells must first be lysed before the polypeptide is recovered.

herg4 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to diagnostic reagents comprising herg4 polynucleotides or fragments thereof, and their use in a diagnostic assay. It also relates to diagnostic kits containing such reagents for the detection of a mutated form of herg4 gene containing a polynucleotide of the invention. Detection of a mutated form of herg4 gene associated with a dysfunction will provide a diagnostic tool that can add to or define a diagnosis of a disease or susceptibility to a disease which results from under-expression, over-expression or altered expression of herg4. Individuals carrying mutations in the herg4 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled herg4 nucleotide sequences, such as described in Picketts et al, 1992, Human Genetics 89: 155-157.

In another embodiment, perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising herg4 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The invention also relates to a diagnostic kit for the detection of a disease or a susceptibility to a disease in a subject related to expression or activity of herg4 polypeptide comprising an amino acid sequence which is at least 80% identical to the amino acid sequence of SEQ ID NO: 2 over its entire length in a subject containing a monoclonal or polyclonal antibody specific to herg4 polypeptide, or a fragment thereof provided this fragment crossreact with monoclonal or polyclonal antibodies specific to herg4 polypeptide.

The diagnostic assays offer a process for diagnosing or determining a susceptibility to epilepsy, migraine, gliomas, abnormal proliferation and cancer, through detection of mutation in the herg4 gene by the methods described.

In addition, epilepsy, migraine, gliomas, abnormal proliferation and cancer, can be diagnosed by methods comprising determining from a sample derived from a subject an abnormally decreased or increased level of herg4 polypeptide or herg4 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as a herg4, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the herg4 polypeptides. The term "immunospecific" means that the antibodies have substantial greater affinity for the polypeptides of the invention than their affinity for other related polypeptides in the prior art.

Antibodies generated against the herg4 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against herg4 polypeptides may also be employed to treat cerebral and cardiac and renal ischemias, brain and cardiac diseases, inflammation, pain, to mimic or antagonize effect of endogenous neurotransmitters and hormones, or to diagnose or treat any disorder related to abnormal expression of said herg4 polypeptides, among others.

### Vaccines/immunological products

Another aspect of the invention relates to a method for inducing an immunological response in a mammal which comprises inoculating the mammal with herg4 polypeptide, or a fragment thereof, adequate to produce antibody and/or T cell immune response to protect said animal from epilepsy, migraine, to alter or prevent effect of endogenous neurotransmitters and hormones, or to diagnose or treat any disorder related to abnormal expression of the herg4 polypeptide, among others. Yet another aspect of the invention relates to a method of inducing immunological response in a mammal which comprises, delivering herg4 polypeptide via a vector directing expression of herg4 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a herg4 polypeptide wherein the composition comprises a herg4 polypeptide or herg4 gene. The vaccine formulation may further comprise a suitable carrier. Since herg4 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The herg4 polypeptide of the present invention may be employed in a screening process for compounds which bind the channel and which activate (agonists) or inhibit activation of (antagonists) the channel polypeptide of the present invention. The present invention also relates to screening assay for identifying agonists to herg4 polypeptide comprising an amino acid sequence which is at least 80% identical to the amino acid sequence of SEQU ID NO: 2 over its entire length or a fragment thereof comprising:
(a) contacting host cells according to the invention with a candidate compound; and
(b) determining whether the candidate compound effects a signal generated by activation of the herg4 polypeptide.

It also relates to screening assay for identifying antagonists to herg4 polypeptide comprising an amino acid sequence which is at least 80% identical to the amino acid sequence of SEQ ID NO: 2 over its entire length or a fragment thereof comprising:
(a) contacting said cell according to the invention with an agonist; and
(b) determining whether the signal generated by said agonist is diminished in the presence of a candidate compound.

Agonists and antagonists identified by such screening asays are encompassed in the scope of the invention.

Thus, polypeptides of the invention or their fragments may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al., Current Protocols in Immunology* 1(2):Chapter 5 (1991).

herg4 polypeptides are implicated in many biological functions, and possibly pathologies. Accordingly, it is desirous to find compounds and drugs which stimulate herg4 on the one hand and which can inhibit the function of herg4 on the other hand. In general, agonists are employed for therapeutic and prophylactic purposes for such conditions as to alter effect of endogenous neurotransmitters and hormones. Antagonists may be employed for a variety of therapeutic and prophylactic purposes for such conditions as epilepsy (hyperexitability), migraines, abnormal proliferation and cancer, to antagonize effect of endogenous neurotransmitters and hormones.

In general, such screening procedures involve producing appropriate cells which express the receptor polypeptide of the present invention on the surface thereof. Such cells include cells from mammals, yeast, *Drosophila* or *E. coli.* Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the channel is detected by means of a label directly or indirectly associated with the candidate compound or in an assay involving competition with a labeled competitor. Further, these assays may test whether the candidate compound results in a signal generated by activation of the channel, using detection systems appropriate to the cells bearing the channel at their surfaces. Inhibitors of activation are generally assayed in the presence of a known agonist and the effect on activation by the agonist by the presence of the candidate compound is observed. Standard methods for conducting such screening assays are well understood in the art.

The recording of herg4 channel activity may be carried out either by membrane voltage analysis of transfected cells or microinjected xenope oocytes, directly (patch-clamp for example) or indirectly (fluorescent probes sensitive to changes of membrane potential). The assays may simply test binding of a candidate compound wherein adherence to the cells bearing the channel is detected by means of a label directly or indirectly associated with the candidate compound or in an assay involving competition with a labeled competitor. Further, these assays may test whether the candidate compound results in a signal generated by activation of the channel, using detection systems appropriate to the cells bearing the channel at their surfaces. Inhibitors of activation are generally assayed in the presence of a known agonist and the effect on activation by the agonist by the presence of the candidate compound is observed. Standard methods for conducting such screening assays are well understood in the art.

Examples of potential herg4 antagonists include antibodies or, in some cases, oligonucleotides or proteins which are closely related to the ligand of the herg4, e.g., a fragment of the ligand, or small molecules which bind to the receptor but do not elicit a response, so that the activity of the channel is prevented.

### Prophylactic and Therapeutic Methods

This invention provides methods of treating abnormal conditions related to both an excess of and insufficient amounts of herg4 activity.

It also relates to therapeutic compositions for the treatment of a subject in need of enhanced activity or expression of herg4 polypeptide, containing:
(a) a therapeutically effective amount of an agonist of herg4 polypeptide according to the present invention, and/or
(b) a polynucleotide according to the invention in a form so as to effect production of said herg4 polypeptide activity *in vivo*, or a DNA or RNA vectors or a host cell according to the invention.

If the activity of herg4 is in excess, several approaches are available. One approach comprises administering to a subject an inhibitor compound (antagonist) as hereinabove described along with a pharmaceutically acceptable carrier in an amount effective to inhibit activation by blocking binding of ligands to the herg4, or by inhibiting a second signal, and thereby alleviating the abnormal condition.

In another approach, soluble forms of herg4 polypeptides still capable of binding the ligand in competition with endogenous herg4 may be administered. Typical embodiments of such competitors comprise fragments of the herg4 polypeptide.

In still another approach, expression of the gene encoding endogenous herg4 can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al*., *Science* (1988) 241:456; Dervan *et al., Science* (1991) 251:1360. These oligomers can be administered *per se* or the relevant oligomers can be expressed *in vivo*.

The invention also relates to a therapeutic composition for the treatment of a subject having need to inhibit activity or expression of herg4 polypeptide, containing:
(a) a therapeutically effective amount of an antagonist of herg4 polypeptide according to the present invention, and/or
(b) a nucleic acid molecule that inhibits the expression of the nucleotide sequence encoding said herg4 polypeptide, and/or
(c) a therapeutically effective amount of a polypeptide that competes with said herg4 polypeptide.

For treating abnormal conditions related to an under-expression of herg4 and its activity, several approaches are also available. One approach comprises administering to a subject a therapeutically effective amount of a compound which activates herg4, i.e., an agonist as described above, in combination with a pharmaceutically acceptable carrier, to thereby alleviate the abnormal condition. Alternatively, gene therapy may be employed to effect the endogenous production of herg4 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells in vivo and expression of the polypeptide in vivo. For overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996).

### Formulation and Administration

Peptides, such as the soluble form of herg4 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

### Cloning the Human herg4 cation Channel

The sequence of the herg4 cation channel was first identified by searching a database containing approximately 2 million human ESTs, which was generated using high throughput automated DNA sequence analysis of randomly selected human cDNA clones (Adams, M.D. *et al., Nature* 377:3-174 (1995); Adams, M.D. *et al., Nature 355*:632-634 (1992); and Adams, M.D. *et al., Science 252*:1651-1656 (1991)). Sequence homology comparisons of each EST were performed against the GenBank database using the blastn and tblastn algorithms (Altschul, S.F. et al., *J. Mol. Biol. 215*:403-410 (1990)). A specific homology search using a cAMP binding domain of a cyclic nucleotide gated channel (CNG) amino acid sequence against this human EST database revealed one EST, from a cerebellum cDNA library, with approximatively 60% similarity to CNG3. The sequence comparison suggested that it contained the partial open reading frame of a new protein. Sequence of the gene was confirmed by double strand DNA sequencing using the TaqFs (Perkin Elmer) and the gene was shown to be completely new by a blast search against Genbank release 110. The entire herg4 coding region was obtained with a combination of 5' sequence finding techniques and fragments were combined by recombinant DNA techniques,it was then amplified by PCR and inserted into the expression vector pCR2.1 (Invitrogen).

### Example 2

### Cloning and Expression of herg4 in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3 (Invitrogen). Mammalian host cells that could be used include, human HEK 293, H9 and Jurkat cells, mouse NIH3T3 and C127 cells, Cos 1, Cos 7 and mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem. J. 227*:277-279 (1991); Bebbington *et al., Bio/Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pCMVsport3.0 contains the strong promoter (CMV) of the Cytomegalovirus. Multiple cloning sites, e.g., with the restriction enzyme cleavage site EcoRI, facilitate the cloning of the gene of interest.

### Example 3

### Tissue distribution of herg4 mRNA expression

Northern blot analysis can be carried out to examine herg4 gene expression in human tissues, using methods described by, among others, Sambrook *et al.,* cited above. A cDNA probe containing the entire nucleotide sequence of the herg4 protein can be labeled with ³²P using the Rediprime™ DNA labeling system (Amersham Life Science, Arlington, IL), according to manufacturer's instructions. After labeling, the probe can be purified using a CHROMA SPIN- 100™ column (Clontech Laboratories, Inc.), according to manufacturer's protocol number PT1200-1. The purified labeled probe was then used to examine various human tissues for herg4 mRNA.

Multiple Tissue Northern (MTN) blots containing various human tissues can be obtained from Clontech and examined with the labeled probe using ExpressHyb™ hybridization solution (Clontech) according to manufacturer's protocol number PT1190-1. Following hybridization and washing, the blots can be mounted and exposed to film at -70°C overnight, and films developed according to standard procedures.

It will be clear that the invention may be practiced otherwise than as particularly described in the foregoing description and examples.

Numerous modifications and variations of the present invention are possible in light of the above teachings and, therefore, are within the scope of the appended claims.

The entire disclosure of all publications (including patents, patent applications, journal articles, laboratory manuals, books, or other documents) cited herein are incorporated by reference.

## Claims

1. An isolated polynucleotide comprising a nucleotide sequence that has at least 80% identity to a nucleotide sequence encoding the herg4 polypeptide of SEQ ID NO:2 over its entire length; or a nucleotide sequence complementary to said nucleotide sequence at least 80 % of said nucleotide sequence.

2. The polynucleotide of claim 1 which is DNA or RNA.

3. A polynucleotide according to one of claims 1 and 2 wherein said nucleotide sequence is at least 80% identical to that contained in SEQ ID NO:1.

4. A polynucleotide according to one of claims 1 and 3 wherein said nucleotide sequence comprises the herg4 polypeptide encoding sequence contained in SEQ ID NO:1.

5. A polynucleotide according to one of claims 1 and 4 which is polynucleotide of SEQ ID NO: 1.

6. An isolated herg4 polynucleotide comprising a nucleotide sequence selected from the group consisting of :
(a) a nucleotide sequence having at least 80% identity to a nucleotide sequence encoding the herg4 polypeptide expressd by the cDNA insert deposited at the ATCC with Deposit Number 203078 ; and
(b) a nucleotide sequence complementary to the nucleotide sequence of (a).

7. A nucleic acid probe for the detection of a polynucleotide according to claim 1 containing all or part of the complementary sequence of SEQ ID No. 1.

8. A DNA or RNA molecule comprising an expression system, or vectors, wherein said expression system is capable of producing a herg4 polypeptide comprising an amino acid sequence, which has at least 80% identity with the polypeptide of SEQ ID NO:2 when said expression system is present in a compatible host cell.

9. A host cell comprising the expression system of claim 8.

10. A herg4 polypeptide comprising an amino acid sequence which is at least 80% identical to the amino acid sequence of SEQ ID NO:2 over its entire length.

11. The polypeptide of claim 11 which comprises the amino acid sequence of SEQ ID NO:2.

12. An antibody immunospecific for the herg4 polypeptide of claim 11.

13. Diagnostic kit for the detection of a mutated form of herg 4 gene containing a polynucleotide according to anyone of claims 1 to 7.

14. Therapeutic composition for the treatment of a subject in need of enhanced activity or expression of herg4 polypeptide, containing:
(a) a therapeutically effective amount of an agonist of herg4 polypeptide of claim 10, and/or
(b) a polynucleotide according to one of claims 1 to 6 in a form so as to effect production of said herg4 polypeptide activity *in vivo*, or a DNA or RNA according to claim 8 or a host cell according to claim 9.

15. Therapeutic composition for the treatment of a subject having need to inhibit activity or expression of herg4 polypeptide, containing:
(a) a therapeutically effective amount of an antagonist of herg4 polypeptide of claim 10, and/or
(b) a nucleic acid molecule that inhibits the expression of the nucleotide sequence encoding said herg4 polypeptide, and/or
(c) a therapeutically effective amount of a polypeptide that competes with said herg4 polypeptide.

16. Diagnostic kit for the detection of a disease or a susceptibility to a disease in a subject related to expression or activity of herg4 polypeptide of claim 10 in a subject containing a monoclonal or polyclonal antibody specific to herg 4 polypeptide.

17. Screening assay for identifying agonists to herg4 polypeptide of claim 10 comprising:
(a) contacting host cells according to claim 9 with a candidate compound; and
(b) determining whether the candidate compound effects a signal generated by activation of the herg4 polypeptide.

18. An agonist identified by the assay of claim 17.

19. Screening assay for identifying antagonists to herg4 polypeptide of claim 10 comprising:
(a) contacting said cell according to claim 9 with an agonist; and
(b) determining whether the signal generated by said agonist is diminished in the presence of a candidate compound.

20. An antagonist identified by the assay of claim 19.
